# EUROPEAN PATENT SPECIFICATION

(11) **EP 3 723 721 B1**
(45) Date of publication and mention of the grant of the patent: **15.09.2021**
(21) Application number: 18833526.9
(22) Date of filing: 12.12.2018
(51) Int. Cl.: A61K 8/64, A61K 8/25, A61Q 11/00

(54) **COMPLEX OF LACTOFERRIN AND SILICA, PROCESS FOR PREPARING AND COMPOSITIONS FOR ORAL HYGIENE**
KOMPLEX AUS LACTOFERRIN UND KIESELSÄURE, VERFAHREN ZUR HERSTELLUNG UND ZUSAMMENSETZUNGEN FÜR DIE MUNDHYGIENE
COMPLEXE DE LACTOFERRINE ET DE SILICE, PROCÉDÉ DE PRÉPARATION ET COMPOSITIONS POUR L'HYGIÈNE BUCCALE

(30) Priority: 13.12.2017 IT 201700143342
(43) Date of publication of application: 21.10.2020
(73) Proprietor: L.I.CO.RICE S.R.L., 55100 Lucca (LU) (IT)
(72) Inventor: CISTERNAS CISTERNAS, Jasna Isabel, 40017 San Giovanni In Persiceto (BO) (IT)
(74) Representative: Bottero, Carlo
(86) International application number: PCT/IB2018/059937
(87) International publication number: WO 2019/116258

(56) References cited:
- EP-A1- 3 192 495
- EP-A1- 3 192 498
- WO-A1-2016/193877
- DE-A1-102010 063 720
- US-A1- 2003 003 059
- Evonik ET AL: "Aerosil 300", , 31 March 2013 (2013-03-31), XP055483419, Retrieved from the Internet: URL:https://www.palmerholland.com/Assets/C E/Documents/data-sheets/AEROSIL%20300.pdf [retrieved on 2018-06-12]
- Evonik: "Product Overview-AEROSIL?", , 1 October 2015 (2015-10-01), pages 1-19, XP055367439, Retrieved from the Internet: URL:http://www.aerosil.com/sites/lists/RE/ DocumentsSI/AEROSIL-product-overview-EN.pd f [retrieved on 2017-04-25]

## Description

The present invention relates to a complex comprising one lactoferrin and one silica, process for the production thereof, and a composition thereof for oral hygiene, in particular in cases of increased oxidative stress of the oral cavity.

It is well known that oral hygiene is a determining factor in the prevention of diseases of the dental apparatus, such as caries, gingivitis, periodontitis, peri-implantitis, etc.

Periodontitis is known to be a chronic inflammatory disease characterized by the destruction of the tooth's support structures (periodontal ligament, alveolar bone). Periodontitis has a very high prevalence in the general population (around 10-15% of adults) and induces numerous negative effects on quality of life.

On the other hand, peri-implantitis is an inflammation process that affects the tissue surrounding an implant osseo-integrated implant, leading to a loss in the bone tissue and therefore in the implant itself. The cause can be infectious, or it can be due to incorrect implant-prosthetic passivation (occlusal trauma), although both causes can be clinically found. Radiographic examination makes it possible to easily distinguish peri-implantitis from occlusal trauma from one caused by bacteria.

To prevent or slow down the development of these pathologies, particularly accurate oral hygiene becomes critical in people suffering from dysfunctions or diseases, in particular diabetes, which increases oxidative stress in the oral cavity.

From a clinical point of view, diabetes mellitus is a clinically and genetically heterogeneous group of metabolic disorders characterized by high levels of glucose in the blood (hyperglycaemia). Hyperglycaemia can be a direct consequence of a deficit of insulin secretion caused by the dysfunction of the pancreatic β cells or by resistance to the same hormone by the hepato-muscular tissues, or also a combination of the aforementioned phenomena. This metabolic disorder is frequently associated with an alteration of the metabolism of adipocytes and can by all means be considered a syndrome. In the long-term, chronic hyperglycaemia causes multiple damage to different organs such as, for instance, heart, eyes, kidneys, nerves, vascular system.

Numerous recent epidemiological studies confirm that diabetes can be considered an important risk factor for the development of periodontal disease. The scientific evidence of the last 15-20 years supports a significant independent association between periodontal inflammation and glycaemic state. The susceptibility to the onset of periodontitis is three times greater in patients with diabetes mellitus.

Recent data provided by the World Health Organization shows that the incidence of the diabetic pathology is dramatically increasing. A direct consequence of this data is the parallel increase in patients suffering from periodontal disease, since diabetes is a risk factor for the development of periodontitis, regardless of the reduction in smoking habits and improved oral hygiene.

Diabetes mellitus and periodontal disease are both characterized by a state of systemic inflammation. Chronic hyperglycaemia, as well as periodontitis, induce the hyper-activation of the immune system, resulting in the hyper-production of pro-inflammatory molecules (TNF-a, IL6, IL1). Entering the systemic circle, these can negatively affect the organism. In this sense, chronic inflammation of the gingival tissues can worsen the glycaemic and clinical profile of a patient suffering from diabetes mellitus. This relationship must therefore be considered with a bidirectional nature, in light of the foregoing.

Another aspect related to chronic periodontitis is the alteration of the immune system in terms of the balance between antioxidant agents and those reacting to oxygen, in favour of the latter. Oxidative stress can aggravate the clinical manifestations of a diabetic patient. On the other hand, diabetes is in turn a cause of oxidative stress and this can justify the fact that the clinical scenarios deriving from periodontitis are particularly aggressive in diabetic patients. The high prevalence and severity of periodontal disease in diabetic patients can be considered a complication resulting from a state of chronic hyperglycaemia and an accumulation of advanced glycation end-products (AGEs). The protracted oxidative stress in the gingival tissues could justify the aggressive destruction of periodontal tissues found in diabetic patients. The interaction between AGEs and their cell receptors induces an increase in oxidative stress that can contribute to chronic monocyte hyper-activation, to activation of the NF-Kb system resulting in the expression of pro-inflammatory mRNAs.

Lactoferrin is an antimicrobial and iron-carrying glycoprotein, naturally present in various biological fluids of mammals, such as milk, saliva, tears. It is also present in neutrophil granulocytes, which are immune cells with functions of defence against bacterial and fungal infections.

Lactoferrin is considered a particularly useful product for its antioxidant, immunomodulatory and anti-infective properties. The antimicrobial properties of lactoferrin are mainly due to its ability to bind iron, subtracting it from the metabolism of those bacterial species, such as *Escherichia coli,* which depend on it for its own multiplication and adhesion to the intestinal mucosa (bacteriostatic effect). It also has a direct antibacterial action (bactericidal), thanks to the ability to damage the outer layers of the cell membrane of some gram-negative bacterial species.

The use of lactoferrin in the field of oral care products has currently not been widely used, mainly due to its poor stability and compatibility with the excipients normally used in oral care products, such as toothpastes, gels or mouthwashes. Lactoferrin also has a relatively low bio-availability when applied topically and is subject to oxidative degradation phenomena. Lactoferrin-comprising oral care compositions are disclosed e.g. in US2003/003056 A1, EP3192498 A1, EP 3192495 A1 and DE 102010063720.

The Applicant has therefore posed the problem of obtaining a composition containing lactoferrin which is suitable for use in oral hygiene, in particular in the form of toothpaste, gel or mouthwash, and which has high stability and bio-availability, so as to perform an antibacterial and antioxidant action within the oral cavity that is effective and long-lasting.

This problem and others which will be better illustrated below have been solved by the association of one lactoferrin with at least one silica having a high specific surface area, which is able to complex with the protein to form a stable complex which increases and preserves the long antioxidant and antibacterial activity of lactoferrin.

According to a first aspect, the present invention therefore relates to a complex of at least one lactoferrin and at least one silica having a specific surface area comprised between 100 m²/g and 1000 m²/g, preferably between 150 m²/g and 700 m²/g (BET area, measured according to standard ISO 9277-2010), said complex being obtainable by adding said lactoferrin to said silica in the form of a sol-gel with an aqueous solvent, and subsequent drying of the sol-gel.

Preferably, the drying is carried out through treatment with supercritical CO₂ or by lyophilization, as better illustrated below.

According to another aspect, the present invention relates to a composition for oral hygiene, in particular for the oral hygiene of diabetic patients, which comprises said complex and at least one physiologically acceptable excipient.

According to another aspect, the present invention relates to a complex as defined above for use, through local application within oral cavity, in the prevention or treatment of diseases of dental apparatus, in particular for use in the prevention or treatment of periodontitis or peri-implantitis, preferably in patients with diabetes.

As for lactoferrin, this can be of natural or synthetic origin, and can include different forms, both monomeric and oligomeric, possibly already bound to iron (ololactoferrin) or not bound to iron (apolactoferrin).

As regards silica, this can be obtained through synthesis or through the processing of materials of natural origin, as better illustrated below. Among the synthetic silicas, particularly preferred forms are fumed silicas or precipitated silicas. Preferably, the silica is a hydrophilic silica.

For the purposes of the present invention, silicas in the form of aerogel are particularly preferred. These are amorphous silicas characterized by a high specific surface area, and also by a very high porosity and very low density. The porosity is generally greater than 85% and can even reach values of 95-99%, and the density is typically from 0.003 g/cm³ to 0.8 g/cm³, preferably from 0.005 g/cm³ to 0.3 g/cm³.

A silica in the form of aerogel obtained from vegetable waste is particularly preferred, in particular from husk resulting from the processing of grains, such as rice, oats, wheat, rye, spelt. This silica, which is obtained from renewable sources, features a high level of bio-compatibility and has a high surface area and a highly porous, open-pore structure, which favours the interaction with lactoferrin. The silica obtained from rice husk is particularly preferred.

The silica in the form of aerogel from vegetable waste can be obtained through a multi-stage process that comprises: combustion of vegetable waste, processing of the ashes thus obtained with an alkaline solution (NaOH) to obtain a (sodium) silicate, processing of the latter with an acid solution (HCl) with the formation of a wet sol-gel, and finally drying of the wet sol-gel, preferably by treatment with supercritical CO₂. Further details on the preparation of such silica forms are reported, for example, in patent application WO 2016/193877 and in the article by R.S. Kumar et al. Int. J. Chem. Eng. and Appls., Vol. 4, No. 5, October 2013.

Preferably, in the complex according to the present invention, said at least one lactoferrin and said at least one silica are present in a weight ratio of from 0.05:1 to 10:1, more preferably from 0.1:1 to 5:1.

Preferably, the composition according to the present invention comprises:
from 10% to 70% by weight of at least one silica;
from 0.05% to 15% by weight of at least one lactoferrin;
from 25% to 80% by weight of an aqueous phase.

More preferably, the composition according to the present invention comprises:
from 20% to 50% by weight of at least one silica;
from 0.2% to 10% by weight of at least one lactoferrin;
from 40% to 75% by weight of an aqueous phase.

It should be noted that the silica amounts indicated above refer to the amount of overall silica present in the composition, whether it is complexed or not complexed with lactoferrin.

According to a preferred embodiment, the composition according to the present invention comprises the complex of at least one lactoferrin and at least one silica as defined above in mixture with a hydrophilic silica that has not been complexed with lactoferrin, in particular a synthetic silica, preferably a hydrophilic fumed silica. The addition of a non-complexed silica has the main purpose of further stabilizing the silica and lactoferrin complex, substantially acting as a barrier to the interaction with other ingredients of the composition that could destabilize the complex itself.

The weight ratio of silica complexed with lactoferrin and hydrophilic silica not complexed with lactoferrin is preferably between 1:200 and 1:1, more preferably between 1:150 and 1:5.

As for the aqueous phase, this preferably comprises a mixture of water and a polyol, more preferably glycerol or sorbitol. The weight ratio of water to polyol is preferably comprised between 1:20 and 1:1, more preferably between 1:10 and 1:2.

The Applicant has found that the lactoferrin and silica complex according to the present invention makes it possible to obtain an improved antibacterial and antioxidant effect with respect to the lactoferrin used alone with the same concentrations, which is carried out for longer times, so as to prolong the effect without having to repeat the application of the composition too often.

The complex according to the present invention is preferably prepared through a process which comprises:
preparing a silica having a specific surface area ranging from 100 m²/g to 1000 m²/g, preferably from 150 m²/g to 700 m²/g (BET area, measured according to the standard ISO 9277-2010) in a sol-gel form with an aqueous solvent;
adding at least one lactoferrin to the silica in sol-gel form;
subjecting the mixture thus obtained to a drying step.

The addition of the lactoferrin to the silica in a sol-gel form can be carried out through gradual addition of the protein to the sol-gel and mechanical mixing until a homogeneous dispersion is obtained. The mixing temperature is generally maintained between 15°C and 50°C.

Preferably, the drying is carried out by treatment with supercritical CO₂ or by lyophilization. In this way, the complexation of the silica and lactoferrin is guaranteed, without the latter being denatured or undergoing other structural modifications which would compromise its functionality.

The lactoferrin and silica complex according to the present invention can be treated with at least one polyol, preferably glycerol or sorbitol. The polyol has the function of covering the silica particles loaded with the lactoferrin, further stabilizing the structure.

Water can be used as an aqueous solvent, optionally in admixture with at least one water soluble organic solvent, for example ethyl acetate or acetone.

Preferably, the drying step with supercritical CO₂ is carried out in an autoclave, at a temperature from 15°C to 60°C and a pressure from 80 bars to 150 bars. In order to avoid the degradation of the lactoferrin, using a temperature not greater than 60°C is preferable. The treatment is carried out for a time preferably ranging from 2 hours to 10 hours, more preferably from 4 hours to 8 hours.

Preferably, the drying step by means of lyophilization (cryo-drying) is carried out by cooling under vacuum at a temperature between -80°C and -30°C and subsequent heating, again under vacuum, at a temperature not greater than 60°C, preferably between 20°C and 50°C. The pressure during lyophilization is preferably maintained between 0.1 bar and 0.8 bar.

The oral hygiene composition according to the present invention comprises, in addition to the complex, at least one physiologically acceptable excipient. This is in order to obtain a product for oral hygiene in different forms, for example toothpaste, mouthwash, gel, chewing gum, tablets for oral use, gingival dyeing, and the like.

For this purpose, the composition according to the present invention can comprise ingredients commonly used in the field of dental hygiene products, such as for example: abrasive agents in particulate form, humectants, binders, thickeners, viscosity regulators, surfactants, sweeteners, flavourings, preservatives, anti-plaque agents, dyes, etc.

The following embodiment examples are provided for the sole purpose of illustrating the present invention and are not to be intended to limit the scope of protection defined by the appended claims.

### EXAMPLES 1-3.

The compositions shown in Table 1 were prepared (% by weight of the total composition):

**TABLE 1**

| EXAMPLE | 1 | 2 | 3 (*) |
|---|---|---|---|
| glycerol | 51.6 | 51.0 | 74.0 |
| water | 7.0 | 15.0 | 25.0 |
| hydrophilic silica | 40.0 | 30.0 | -- |
| rice husk silica | 0.4 | 3.0 | -- |
| lactoferrin | 1.0 | 1.0 | 1.0 |

| | | | |
|---|---|---|---|
| (*) comparative | | | |

- Hydrophilic silica: hydrophilic fumed silica having a specific surface area of about 200 m²/g (BET area, measured according to standard ISO 9277-2010) (commercial product Aerosil™ 200 by Evonik);
- Rice husk silica: silica in the form of an aerogel having a specific surface area of about 550 m²/g (BET area, measured according to standard ISO 9277-2010) and a density of about 0.050 g/cm³.

The rice husk silica and lactoferrin were previously complexed through the following process. The rice husk silica was prepared as described in Example 1 of WO 2016/193877, with the difference that, after obtaining the silica sol-gel, this was added to the lactoferrin and then subjected to drying with supercritical CO₂ as described in the same Example 1, with the exception that the silica sol-gel added with lactoferrin was treated in the autoclave at a temperature of 50°C and a pressure of 100 bar, for a time equal to 6 hours, in order to obtain the drying of the sol-gel without causing degradation of lactoferrin.

The complex thus obtained and the other ingredients of the composition were mixed in a flask with the aid of a magnetic bar, until a homogeneous formulation was obtained. It should be noted that hydrophilic silica has been added as such, i.e. not complexed with lactoferrin.

Antioxidant and antibacterial properties of these compositions were verified, evaluating variation of efficacy over time.

### Antioxidant activity (scavenger).

Antioxidant activity (scavenger for free radicals) of the above compositions was evaluated by measuring the ability to counteract the formation of free radicals (ROS) in cell cultures of human keratinocytes. For this purpose, keratinocytes were treated with scalar concentrations of the product (1:2 dilutions starting from 1.0 mg/ml) and then exposed to UVA rays at room temperature to stimulate ROS production. The untreated cells represent the negative control. At the same time, a neutral red uptake (NRU) assay was carried out before and after irradiation in order to verify that cell viability did not significantly decrease under the experimental conditions. The results of the NRU test before exposure to UVA rays demonstrated the absence of cell viability inhibition at all concentrations used. Therefore, all the concentrations were taken into consideration for the ROS dosing.

The results obtained by the ROS dosing demonstrated the antioxidant action, expressed as % reduction of ROS with respect to the negative control.

More in detail, the materials and operating conditions used are reported hereinbelow.

Human keratinocytes (Huker) were used, grown in DMEM (Dulbecco's Modified Eagle medium) containing 10% of foetal bovine serum (FBS) and 1% of antibiotics (penicillin and streptomycin), incubated under standard culture conditions (37°C, 5% CO₂). Good cell cultivation practices were applied.

In order to simulate a condition of environmental stress capable of inducing formation of ROS, the cells were subjected to irradiation with UVA rays. The lamp used in the experiments was a sunlight simulator that reproduces the solar spectrum with a constant UVA emission range comprised between 315 nm and 400 nm and with a radiance of 1.7 mW/cm². The emission of UVB is appropriately screened to avoid cytotoxic damage directed to cell cultures.

The effects on ROS production at different irradiation times were evaluated: 4, 8, 12, 16 and 20 min.

Each sample of the tested compositions was dissolved in water and then diluted in a growth medium to the desired final concentrations comprised between 0.0156 and 1.0 mg/ml (1:2 dilutions). 2',7'-dichlorofluorescine diacetate (DCFDA) was used as a fluorimetric tracer, dissolved in dimethylsulfoxide (DMSO) at a concentration of 50 mM and then diluted in the appropriate buffer up to a concentration of use of 250 µM.

Each cell sample (human keratinocytes) was seeded in 96-well plates. Once a semi-confluent monolayer was reached, the cells were washed with phosphate buffer (PBS) and then incubated with the DCFDA solution for 20 min under standard culture conditions. DCFDA was then removed, the cells were washed in PBS, treated with the different concentrations of the tested composition and with the positive control, incubated for 20 min at standard conditions and then irradiated with UVA for 4, 8, 12, 16 and 20 min. At the end of each irradiation period a measurement with a fluorometer was performed at the excitation wavelength of 485 nm and emission wavelength of 530 nm.

In order to verify that the cell viability did not vary significantly in the presence of the composition under examination and in the experimental conditions applied, the neutral red uptake (NRU) assay was performed before and after irradiation of the cells. Neutral red (NR) is a weakly cationic probe that passes through the cell membrane by nonionic passive diffusion and is concentrated in lysosomes, where it binds by electrostatic interactions to anionic sites of the matrix. The uptake of NR depends on the ability of the cells to create pH gradients, determined in turn by ATP production. The NR thus only accumulates in living cells, from which it is extracted with an acidic ethanol solution: the absorbance of the solution, measured at the spectrophotometer, is proportional to cell viability.

After gently removing the medium, the cells were treated with neutral red (NR) (50 µg/ml) and incubated for 3 hours under standard conditions. The cells were subsequently washed with PBS to remove the dye residues and the NR was extracted. The plates were gently agitated for at least 10 min (to facilitate the dissolution of NR) and the absorbance (optical density, OD) was determined through spectrophotometric measurement at 540 nm. The absorbance measured at 540 nm is proportional to the cell viability. The percentages were calculated based on the absorbance values at 540 nm and taking the absorbance of the negative control (untreated cells) as 100%. In the absence of UVA irradiation, the cell viability of the keratinocytes treated with the product was comparable to that of the untreated keratinocytes for all the concentrations tested, therefore all the concentrations were taken into account in the ROS dosages.

### Antimicrobial activity.

Each composition was tested to verify the antimicrobial activity with respect to *E. coli.* An amount equal to 9 g of the composition was added to 1 ml of inoculum of *E. coli* (ATCC 8739) so as to obtain a final charge of about 10⁵ CFU. After increasing contact times (1, 4, 8 and 24 hours), 0.5 ml of the suspension was taken to carry out the plate count (surface spatulation method). The % variation of the bacterial count was therefore determined with respect to the initial one.

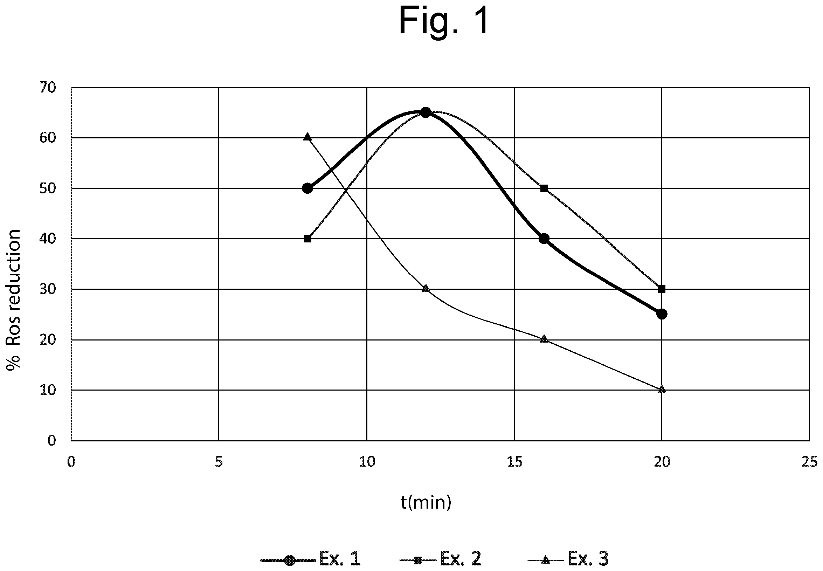

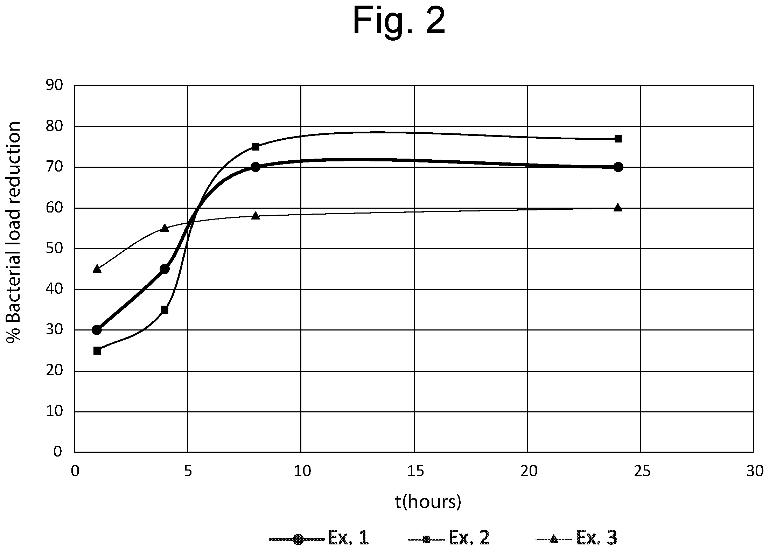

The results are shown in the following tables, where the left column shows the times (in minutes for Table 2 and in hours for Table 3). The same results are reported in the graphs attached as Fig. 1 and Fig. 2.

**TABLE 2**

| (antioxidant effect: % ROS reduction) | | | |
|---|---|---|---|
| EXAMPLE | 1 | 2 | 3 (*) |
| t = 8 | 50 | 40 | 60 |
| t = 12 | 65 | 65 | 30 |
| t = 16 | 40 | 50 | 20 |
| t = 20 | 25 | 30 | 10 |

| | | | |
|---|---|---|---|
| (*) comparative | | | |

**TABLE 3**

| (antibacterial effect: % bacterial load reduction) | | | |
|---|---|---|---|
| EXAMPLE | 1 | 2 | 3 (*) |
| t = 1 | 30 | 25 | 45 |
| t = 4 | 45 | 35 | 55 |
| t = 8 | 70 | 75 | 58 |
| t = 24 | 70 | 77 | 60 |

| | | | |
|---|---|---|---|
| (*) comparative | | | |

From the results obtained, it appears evident that the complex comprising lactoferrin and silica makes it possible to increase efficacy both in terms of antioxidant and antibacterial effect in a more gradual way and maintain them over time, compared to lactoferrin used alone.

### EXAMPLES 4-6.

Examples 2 and 3 were repeated under the same conditions and with the same ingredients shown above (Examples 4 and 5). A further comparative example (Example 6) was also carried out, again with the same procedure and the same ingredients, wherein the lactoferrin was simply mixed with the hydrophilic silica, i.e. without preparing a complex according to the present invention, wherein silica is prepared in a sol-gel form, lactoferrin is added and then dried. The compositions are reported in Table 4 (% by weight of the total composition):

**TABLE 4**

| EXAMPLE | 4 | 5 (*) | 6 (*) |
|---|---|---|---|
| glycerol | 51.0 | 74.0 | 51.0 |
| water | 15.0 | 25.0 | 15.0 |
| hydrophilic silica | 30.0 | -- | 33.0 |
| rice husk silica | 3.0 | -- | -- |
| lactoferrin | 1.0 | 1.0 | 1.0 |

| | | | |
|---|---|---|---|
| (*) comparative | | | |

Antioxidant and antibacterial properties of these compositions were verified, evaluating the variation of the efficacy over time, as described in Examples 1-3.

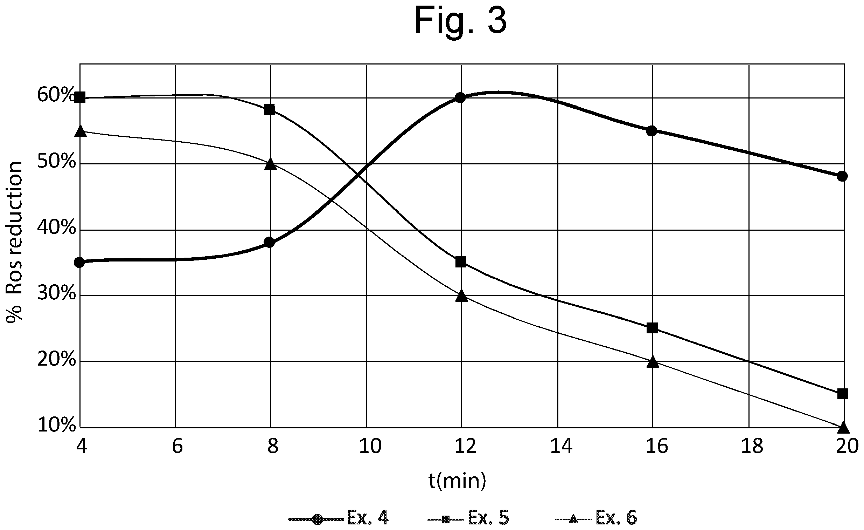

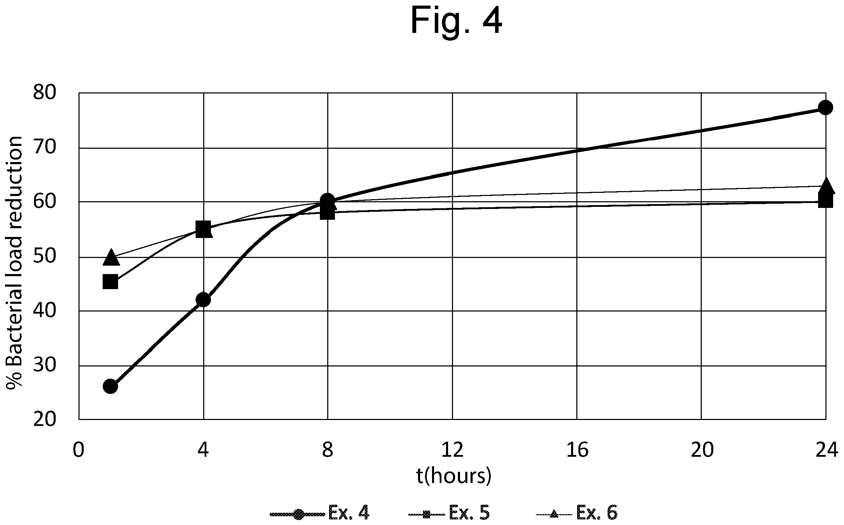

The results are reported in the following tables, where the left column shows the times (in minutes for Table 5 and in hours for Table 6). The same results are reported in the graphs attached as Fig. 3 and Fig. 4.

**TABLE 5**

| (antioxidant effect:% ROS reduction) | | | |
|---|---|---|---|
| EXAMPLE | 4 | 5 (*) | 6 (*) |
| t = 4 | 35 | 60 | 55 |
| t = 8 | 38 | 58 | 50 |
| t = 12 | 60 | 35 | 30 |
| t = 16 | 55 | 25 | 20 |
| t = 20 | 48 | 15 | 10 |

| | | | |
|---|---|---|---|
| (*) comparative | | | |

**TABLE 6**

| (antibacterial effect: % bacterial load reduction) | | | |
|---|---|---|---|
| EXAMPLE | 4 | 5 (*) | 6 (*) |
| t = 1 | 26 | 45 | 50 |
| t = 4 | 42 | 55 | 55 |
| t = 8 | 60 | 58 | 60 |
| t = 24 | 77 | 60 | 63 |

| | | | |
|---|---|---|---|
| (*) comparative | | | |

From the results obtained, it appears evident that the complex comprising lactoferrin and silica makes it possible to increase efficacy both in terms of antioxidant and antibacterial effect in a more gradual way and to maintain them over time, compared to lactoferrin used alone, as well as compared to a simple mixture of lactoferrin with silica, without forming a complex according to the present invention.

## Claims

1. A complex of at least one lactoferrin and at least one silica having a specific surface area comprised between 100 m²/g and 1000 m²/g, preferably between 150 m²/g and 700 m²/g (BET area, measured according to standard ISO 9277-2010), said complex being obtainable by adding said lactoferrin to said silica in the form of a sol-gel with an aqueous solvent, and subsequent drying of the sol-gel.

2. The complex according to any of the previous claims, wherein said at least one silica is a synthetic silica, selected from fumed silicas and precipitated silicas.

3. The complex according to any of the previous claims, wherein said at least one silica is in the form of an aerogel.

4. The complex according to claim 3, wherein said at least one silica in the form of an aerogel has a porosity greater than 85% and a density ranging from 0.003 g/cm³ to 0.8 g/cm³, preferably from 0.005 g/cm³ to 0.3 g/cm³.

5. The complex according to claim 3 or 4, wherein said at least one silica is obtained from vegetable waste, in particular from husk deriving from the processing of grains, preferably rice.

6. The complex according to any of the preceding claims, wherein said at least one lactoferrin and said at least one silica are present in a weight ratio of 0.05:1 to 10:1, preferably from 0.1:1 to 5:1.

7. The complex according to any of the preceding claims, in the form of particles surface treated with at least one polyol, preferably glycerol or sorbitol.

8. A process for preparing a complex according to any of the claims from 1 to 7, which comprises:
preparing a silica having a specific surface area ranging from 100 m²/g to 1,000 m²/g, preferably from 150 m²/g to 700 m²/g (BET area, measured according to the standard ISO 9277-2010) in a sol-gel form with an aqueous solvent;
adding at least one lactoferrin to the silica in a sol-gel form;
subjecting the mixture thus obtained to a drying step.

9. The process according to claim 8, which further comprises treating the dried mixture by adding at least one polyol, preferably glycerol or sorbitol.

10. The process according to claim 8 or 9, wherein the drying step is carried out by treatment with supercritical CO₂.

11. The process according to claim 8 or 9, wherein the drying step is carried out by lyophilization.

12. The process according to claim 10, wherein the drying step with supercritical CO₂ is carried out in an autoclave, at a temperature ranging from 15°C to 60°C and a pressure ranging from 80 bars to 150 bars.

13. The process according to claim 11, wherein the drying step by means of lyophilization is carried out by cooling under vacuum at a temperature between - 80°C and -30°C and subsequent heating, again under vacuum, at a temperature not greater than 60°C, preferably between 20°C and 50°C.

14. The process according to claim 13, wherein the lyophilization is carried out at a pressure between 0.1 bar and 0.8 bar.

15. A composition for oral hygiene, which comprises at least one complex according to any one of claims 1 to 7, and at least one physiologically acceptable excipient.

16. The composition according to claim 15, wherein said at least one complex is in admixture with at least one hydrophilic silica not complexed with lactoferrin, preferably a hydrophilic fumed silica.

17. The composition according to claim 16, comprising:
from 10% to 70% by weight, preferably from 20% to 50% by weight, of at least one silica;
from 0.05% to 15% by weight, preferably from 0.2% to 10% by weight, of at least one lactoferrin;
from 25% to 80% by weight, preferably from 40% to 75% by weight, of an aqueous phase.

18. The composition according to any claim from 15 to 17, wherein the aqueous phase comprises a mixture of water and a polyol, preferably glycerol or sorbitol.

19. A complex according to any of the claims from 1 to 7 for use, through local application within oral cavity, in the prevention or treatment of diseases of dental apparatus, in particular for use in the prevention or treatment of periodontitis or peri-implantitis, preferably in patients with diabetes.

## Patentansprüche

1. Komplex aus mindestens einem Lactoferrin und mindestens einer Kieselsäure aufweisend eine spezifische Oberfläche zwischen 100 m²/g und 1000 m²/g, vorzugsweise zwischen 150 m²/g und 700 m²/g (BET-Fläche, gemessen nach der Norm ISO 9277-2010) enthalten, wobei der Komplex durch Zugabe des Lactoferrins zu der Kieselsäure in Form eines Sol-Gels mit einem wässrigen Lösungsmittel und anschließendes Trocknen des Sol-Gels erhältlich ist.

2. Komplex nach irgendeinem der vorhergehenden Ansprüche, wobei die mindestens eine Kieselsäure eine synthetische Kieselsäure ist, ausgewählt aus pyrogenen Kieselsäuren und gefällten Kieselsäuren.

3. Komplex nach irgendeinem der vorhergehenden Ansprüche, wobei die mindestens eine Kieselsäure in Form eines Aerogels vorliegt.

4. Komplex nach Anspruch 3, wobei die mindestens eine Kieselsäure in Form eines Aerogels eine Porosität von mehr als 85% und eine Dichte im Bereich von 0.003 g/cm³ bis 0.8 g/cm³, vorzugsweise von 0.005 g/cm³ bis 0.3 g/cm³ aufweist.

5. Komplex nach Anspruch 3 oder 4, wobei die mindestens eine Kieselsäure aus Pflanzenabfällen, insbesondere aus Schalen gewonnen wird, die aus der Verarbeitung von Getreiden, vorzugsweise Reis, stammen.

6. Komplex nach irgendeinem der vorhergehenden Ansprüche, wobei das mindestens eine Lactoferrin und die mindestens eine Kieselsäure in einem Gewichtsverhältnis von 0,05:1 bis 10:1, vorzugsweise von 0,1:1 bis 5:1 vorhanden sind.

7. Komplex nach irgendeinem der vorhergehenden Ansprüche in Form von Partikeln, die mit mindestens einem Polyol, vorzugsweise Glycerin oder Sorbit, oberflächenbehandelt sind.

8. Verfahren zur Herstellung eines Komplexes nach irgendeinem der Ansprüche von 1 bis 7, enthaltend:
Vorbereitung einer Kieselsäure aufweisend eine spezifische Oberfläche im Bereich von 100 m²/g bis 1,000 m²/g, vorzugsweise von 150 m²/g bis 700 m²/g (BET-Fläche, gemessen nach der Norm ISO 9277-2010) in Form eines Sol-Gels mit einem wässrigen Lösungsmittel;
Zugabe von mindestens einem Lactoferrin zur Kieselsäure in Form eines Sol-Gels;
Unterziehen der so erhaltenen Mischung einem Trocknungsschritt.

9. Verfahren nach Anspruch 8, das ferner das Behandeln der getrockneten Mischung durch Zugabe von mindestens einem Polyol, vorzugsweise Glycerin oder Sorbit, enthält.

10. Verfahren nach Anspruch 8 oder 9, wobei der Trocknungsschritt durch Behandlung mit überkritischem CO₂ durchgeführt wird.

11. Verfahren nach Anspruch 8 oder 9, wobei der Trocknungsschritt durch Lyophilisierung durchgeführt wird.

12. Verfahren nach Anspruch 10, wobei der Trocknungsschritt mit überkritischem CO₂ in einem Autoklaven bei einer Temperatur im Bereich von 15°C bis 60°C und einem Druck im Bereich von 80 bar bis 150 bar durchgeführt wird.

13. Verfahren nach Anspruch 11, wobei der Trocknungsschritt mittels Lyophilisierung durch Abkühlen unter Vakuum bei einer Temperatur zwischen - 80°C und -30°C und anschließendes Erhitzen, wiederum unter Vakuum, auf eine Temperatur nicht über 60°C, vorzugsweise zwischen 20°C und 50°C, durchgeführt wird.

14. Verfahren nach Anspruch 13, wobei die Lyophilisierung bei einem Druck zwischen 0,1 bar und 0,8 bar durchgeführt wird.

15. Zusammensetzung für Mundhygiene, die mindestens einen Komplex nach irgendeinem der Ansprüche von 1 bis 7 und mindestens einen physiologisch verträglichen Hilfsstoff enthält.

16. Zusammensetzung nach Anspruch 15, wobei der mindestens eine Komplex mit mindestens einer hydrophilen Kieselsäure vermischt ist, die nicht mit Lactoferrin komplexiert ist, vorzugsweise eine hydrophile pyrogene Kieselsäure.

17. Zusammensetzung nach Anspruch 16, enthaltend:
von 10 bis 70 Gew.-%, vorzugsweise von 20 bis 50 Gew.-% mindestens einer Kieselsäure;
von 0,05 bis 15 Gew.-%, vorzugsweise von 0,2 bis 10 Gew.-% mindestens eines Lactoferrins;
von 25 bis 80 Gew.-%, vorzugsweise von 40 bis 75 Gew.-% einer wässrigen Phase.

18. Zusammensetzung nach irgendeinem der Ansprüche von 15 bis 17, wobei die wässrige Phase eine Mischung aus Wasser und einem Polyol, vorzugsweise Glycerin oder Sorbit, enthält.

19. Komplex nach irgendeinem der Ansprüche 1 bis 7 zur Verwendung, durch lokale Anwendung in der Mundhöhle, bei der Vorbeugung oder Behandlung von Erkrankungen des Zahnapparates, insbesondere zur Verwendung bei der Vorbeugung oder Behandlung von Parodontitis oder Periimplantitis, vorzugsweise bei Patienten mit Diabetes.

## Revendications

1. Complexe d'au moins une lactoferrine et au moins une silice ayant une aire de surface spécifique comprise entre 100 m²/g et 1000 m²/g, de préférence entre 150 m²/g et 700 m²/g (aire BET, mesurée selon le standard ISO 9277-2010), ledit complexe pouvant être obtenu en ajoutant ladite lactoferrine à ladite silice sous forme d'un sol-gel avec un solvant aqueux, et séchage successif du sol-gel.

2. Complexe selon l'une quelconque des revendications précédentes, dans lequel ladite au moins une silice est une silice synthétique, sélectionnée parmi des silices pyrogénées et des silices précipitées.

3. Complexe selon l'une quelconque des revendications précédentes, dans lequel ladite au moins une silice est sous forme d'un aérogel.

4. Complexe selon la revendication 3, dans lequel ladite au moins une silice sous forme d'un aérogel a une porosité supérieure à 85 % et une densité allant de 0,003 g/cm³ à 0,8 g/cm³, de préférence de 0,005 g/cm³ à 0,3 g/cm³.

5. Complexe selon la revendication 3 ou 4, dans lequel ladite au moins une silice est obtenue à partir de déchets végétaux, en particulier à partir d'enveloppe dérivant de la transformation de céréales, de préférence de riz.

6. Complexe selon l'une quelconque des revendications précédentes, dans lequel ladite au moins une lactoferrine et ladite au moins une silice sont présentes dans un rapport pondéral de 0,05:1 à 10:1, de préférence de 0,1:1 à 5:1.

7. Complexe selon l'une quelconque des revendications précédentes, sous la forme de surface de particules traitée avec au moins un polyol, de préférence du glycérol ou sorbitol.

8. Procédé de préparation d'un complexe selon l'une quelconque des revendications de 1 à 7, qui comprend :
préparer une silice ayant une aire de surface spécifique allant de 100 m²/g à 1000 m²/g, de préférence de 150 m²/g à 700 m²/g (aire BET, mesurée selon le standard ISO 9277-2010) sous forme de sol-gel avec un solvant aqueux ;
ajouter au moins une lactoferrine à la silice sous forme de sol-gel ;
soumettre le mélange ainsi obtenu à une étape de séchage.

9. Procédé selon la revendication 8, qui comprend en outre traiter le mélange séché en ajoutant au moins un polyol, de préférence du glycérol ou sorbitol.

10. Procédé selon la revendication 8 ou 9, dans lequel l'étape de séchage est effectuée par traitement avec du CO₂ supercritique.

11. Procédé selon la revendication 8 ou 9, dans lequel l'étape de séchage est effectuée par lyophilisation.

12. Procédé selon la revendication 10, dans lequel l'étape de séchage avec du CO₂ supercritique est effectuée dans un autoclave, à une température allant de 15 °C à 60 °C et à une pression allant de 80 bars à 150 bars.

13. Procédé selon la revendication 11, dans lequel l'étape de séchage au moyen de lyophilisation est effectuée par refroidissement sous vide à une température entre -80 °C et -30 °C et réchauffement successif, à nouveau sous vide, à une température non supérieure à 60 °C, de préférence entre 20 °C et 50 °C.

14. Procédé selon la revendication 13, dans lequel la lyophilisation est effectuée à une pression entre 0,1 bars et 0,8 bars.

15. Composition pour l'hygiène buccale, qui comprend au moins un complexe selon l'une quelconque des revendications 1 à 7, et au moins un excipient physiologiquement acceptable.

16. Composition selon la revendication 15, dans laquelle ledit au moins un complexe est en mélange avec au moins une silice hydrophile non complexée avec lactoferrine, de préférence une silice pyrogénée hydrophile.

17. Composition selon la revendication 16, comprenant :
de 10 % à 70 % en poids, de préférence de 20 % à 50 % en poids, d'au moins une silice ;
de 0,05 % à 15 % en poids, de préférence de 0,2 % à 10 % en poids, d'au moins une lactoferrine ;
de 25 % à 80 % en poids, de préférence de 40 % à 75 % en poids, d'une phase aqueuse.

18. Composition selon l'une quelconque des revendications de 15 à 17, dans laquelle la phase aqueuse comprend un mélange d'eau et d'un polyol, de préférence du glycérol ou sorbitol.

19. Complexe selon l'une quelconque des revendications de 1 à 7 pour l'utilisation, à travers une application locale à l'intérieur de la cavité buccale, dans la prévention ou le traitement de maladies de l'appareil dentaire, en particulier pour une utilisation dans la prévention ou le traitement de parodontite ou péri-implantite, de préférence chez des patients diabétiques.
